# EUROPEAN PATENT SPECIFICATION

(11) **EP 1 883 357 B1**
(45) Date of publication and mention of the grant of the patent: **09.04.2014**
(21) Application number: 06770433.8
(22) Date of filing: 15.05.2006
(51) Int. Cl.: A61F 11/00, A61B 17/221

(54) **LASER-RESISTANT SURGICAL DEVICES**
LASERRESISTENTE CHIRURGISCHE VORRICHTUNGEN
DISPOSITIFS CHIRURGICAUX RESISTANTS AU LASER

(30) Priority: 24.05.2005 US 136204
(43) Date of publication of application: 06.02.2008
(73) Proprietor: C.R.Bard, Inc., Murray Hill, NJ 07974 (US)
(72) Inventor: WOLFE, Justin, Lawrenceville, GA 30044 (US); KNAPP, Tracey, E., Lawrenceville, GA 30045 (US)
(74) Representative: HOFFMANN EITLE
(86) International application number: PCT/US2006/018907
(87) International publication number: WO 2006/127330

(56) References cited:
- WO-A2-02/22028
- WO-A2-2005/034772
- US-A- 4 848 339
- US-A- 5 035 694
- US-A- 5 330 482
- US-A- 6 070 813
- US-A1- 2002 068 943
- US-A1- 2002 095 172
- US-A1- 2002 143 360
- US-A1- 2004 199 200
- US-A1- 2005 080 448
- US-B1- 6 368 328

## Description

### FIELD OF THE INVENTION

US-A1-2002/0068943 is concerned with a "Laser-resistant medical retrieval device" and discloses the features of the pre-characterizing part of claim 1 below.

### BACKGROUND

Lasers are used in various surgical procedures within the body. For example, laser lithotriptors are often used to fracture stones within the ureter or kidney or ureter. To cite a further example, lasers are also used to perform incisions within patient vessels.

Such lasers are normally used in proximity to other surgical devices. For instance, a stone may be fractured while held within a basket of a retrieval device or a laser may be used in the vicinity of a guidewire that is positioned within a patient vessel. Although most lasers that are used in surgical procedures have very short focal lengths to provide greater control over which objects will be affected by the laser, it is possible for an emitted laser beam to strike a surgical device within the body.

In many cases, such a laser beam strike can destroy or damage the surgical device. This is particularly true when the portion of the surgical device that is struck is a wire. For example, if a wire of a retrieval device basket is struck, it is possible for the wire to break. In addition to potentially rendering the basket unusable, such a wire break poses a risk of injury to the vessel or cavity in which the basket is used. To cite a further example, if the surgical device that is struck is a guidewire, and the strike causes the guidewire to break in half, both portions of the guidewire must be removed and a new one put in its place.

From the foregoing, it can be appreciated that it would be desirable to have surgical devices that are resistant to laser strikes.

Refractory materials such as titanium, tantalum, tungsten, carbides and ceramics have been used to withstand laser radiation. For example, see US-A-4848339 and US-A-6070813.

### SUMMARY

The invention is defined in claim 1 below. The dependent claims are directed to optional features and preferred embodiments.

Disclosed are laser-resistant surgical devices that include a laser-resistant wire susceptible to a laser beam strike, the wire comprising a high-temperature metal that is highly resistant to thermal shock.

The laser-resistant surgical device can be a retrieval device that includes a handle, a sheath that extends from the handle, the sheath including a distal tip, and a basket that is extendable from the tip of the sheath, the basket including at least one leg that comprises a wire that includes a high-temperature metal.

In a further embodiment, the laser-resistant surgical device is a guidewire that includes a wire that includes a high-temperature metal.

### BRIEF DESCRIPTION OF THE DRAWINGS

The disclosed laser-resistant devices can be better understood with reference to the following drawings. The components in the drawings are not necessarily to scale.
FIG. 1 is a perspective view of an embodiment of a first laser-resistant surgical device.
FIG. 2 is a side view of the laser-resistant surgical device of FIG. 1.
FIG. 3 is a cross-sectional view of a first embodiment of a basket of the laser-resistant surgical device of FIGS. 1 and 2 taken along line S1-S1 in FIG. 2.
FIG. 4 is a cross-sectional view of a second embodiment of the basket of the laser-resistant surgical device of FIGS. 1 and 2 taken along line S1-S1 in FIG. 2.
FIG. 5 is a cross-sectional view of a first embodiment of a wire that can be used to construct the basket shown in FIGS. 1 and 2.
FIG. 6 is deleted.
FIG. 7 is deleted.
FIG. 8 is deleted.
FIG. 9 is a schematic view that depicts grasping of a stone in a patient vessel using the laser-resistant surgical device of FIGS. 1 and 2 for the purpose of fracturing the stone with a laser lithotriptor.
FIG. 10 is a side view of a first embodiment of a second laser-resistant surgical device.
FIG. 11 is a cross-sectional view of the laser-resistant surgical device of FIG. 10 taken along line S2-S2.
FIG. 12 is a side view of a second embodiment of the second laser-resistant surgical device. FIG. 13 is a cross-sectional view of the laser-resistant surgical device of FIG. 12 taken along line S3-S3.

### DETAILED DESCRIPTION

As is described in the foregoing, the use of lasers during surgical procedures can damage or destroy other surgical devices used in the vicinity of the laser. Such damage or destruction can be reduced or even avoided altogether, however, when the surgical devices used in conjunction with the laser are laser resistant. Described in the following are various laser-resistant surgical devices that are designed to withstand inadvertent laser beam strikes. The laser-resistant surgical devices comprise one or more laser-resistant wires. In some embodiments, the laser-resistant wires comprise a refractory metal. In other embodiments, the laser-resistant wires comprise a high-temperature superalloy.

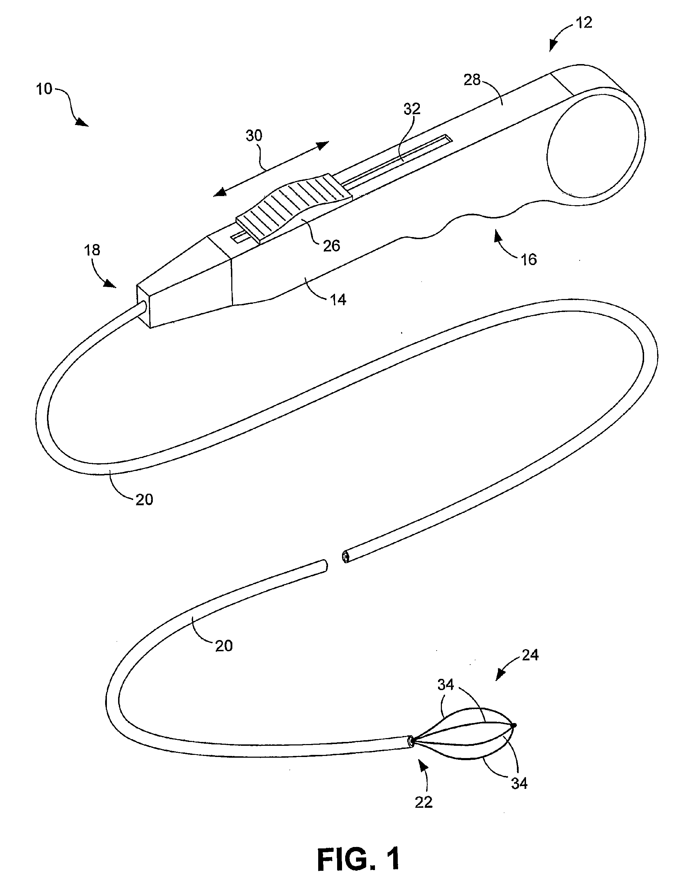

Referring now to the drawings in which like reference numerals identify corresponding components, FIG. 1 illustrates an embodiment of a first laser-resistant surgical device 10. The surgical device 10 comprises a retrieval device suitable for retrieving stones or other objects from a body vessel or cavity, such as a ureter, kidney, or gall bladder. For clarity purposes, the surgical device 10 will be referred to as a retrieval device for the discussion associated with FIGS. 1-9.

As is indicated in FIG. 1, the retrieval device 10 includes a handle 12. The handle 12 includes a handle body 14 that forms a grip 16, which is configured to fit the contours of the operator's hand when the handle is grasped. Extending from a distal end or tip 18 of the handle 12 is an elongated tube or sheath 20 that is adapted for insertion into a patient duct or vessel, such as a urethra. Extending from a distal end or tip 22 of the sheath 20 is a basket 24 that is used to grasp objects within the body, such as stones. The basket 24, which is shown in FIG. 1 in an extended position, is retractable such that the entire basket can be withdrawn into the sheath 20. Such retraction, as well as extension, is achieved through use of a slide 26 that is provided on a top side 28 of the handle 12. More specifically, the slide 26 can be moved back and forth, in the directions indicated by arrow 30, along a slot 32 formed in the top side 28 of the handle 12 to retract and extend the basket 24 relative to the tip 22 of the sheath 20. By way of example, the slide 26 can be moved along the slot 32 using one's thumb. As is further indicated in FIG. 1, the basket 24 includes a plurality of legs 34 that define the basket's shape:

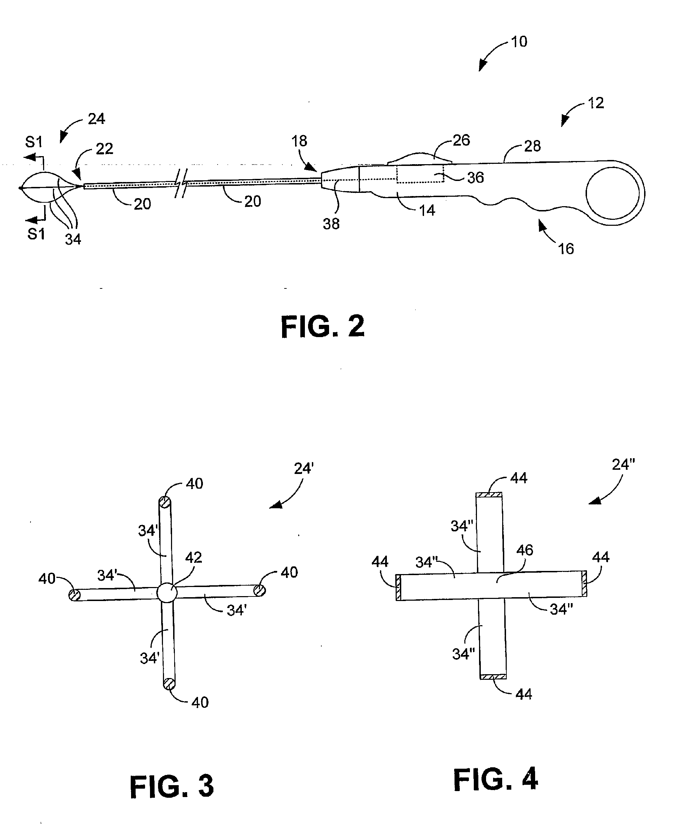

FIG. 2 illustrates the retrieval device 10 from the side. As is indicated in FIG. 2, the slide 26 includes an internal portion 36 to which is secured an internal wire 38 that extends through the sheath 20 to the basket 24. In some embodiments, the internal wire 38 comprises one or more wires or cables that are separate from, but connected to, the legs 34 of the basket 24. hi other embodiments, the internal wire 38 comprises one or more wires that form the legs 34 of the basket 24. In the latter case, one or more of the wires of the basket 24 extend through the sheath 20 to the slide 26. Irrespective of the particular nature of the internal wire 38, forward and rearward movement of the slide 26 along the slot 32 (see FIG. 1) causes similar forward and rearward movement of the internal wire, which causes the basket 24 to be extended from or retracted into the sheath 20. Because the legs 34 of the basket 24 are flexible, they straighten to enable full retraction within the sheath 20. Because the legs 34 have memory, they form the shape of the basket 24 shown in FIGS. 1 and 2 when extended from the sheath 20.

FIGS. 3 and 4 illustrate example constructions for the basket 24 shown in FIGS. 1 and 2. More particularly, FIGS. 3 and 4 illustrate alternative leg arrangements for the basket 24 when the basket is viewed in cross-section taken along line S1-S1 of FIG. 2. Beginning with FIG. 3, illustrated is a first embodiment of a basket 24'. As is indicated in FIG. 3, the basket 24' comprises a plurality of legs 34'. In the embodiment shown in FIG. 3, the basket 24' comprises four such legs 34'. Although four legs 34' are illustrated in FIG. 3 and are described herein, a greater or a fewer number of legs could be used to form the basket 24' depending upon the particular basket arrangement that is desired.

Each leg 34' is formed of a single wire having a round (e.g., circular) cross-section 40. In other words, each leg 34' comprises a single round wire. As is further apparent in FIG. 3, the legs 34' are connected together at a connection point 42. By way of example, this connection point 42 comprises a bond formed using an appropriate bonding method such as welding, soldering, brazing, or applying adhesive or a tie formed with a further wire.

Referring next to FIG. 4, illustrated is a second embodiment of a basket 24". The basket 24" also comprises four legs 34", although greater or fewer legs could be used, as desired. Each leg 34" if formed of a single wire having a rectangular cross-section 44. In other words, each leg 34' comprises a single rectangular wire. In cases in which the sides of the rectangular cross-sections 44 are not all equal to each other, the rectangular wire can comprise a flat wire that is wider than it is thick. Such an arrangement is shown in FIG. 4. With such a configuration, the basket 24" is well suited for dilating a patient vessel, such as a ureter or blood vessel.

As with the basket legs 34' of FIG. 3, the basket legs 34" are connected together at a connection point 46. By way of example, this connection point 42 can comprise a bond formed using an appropriate bonding method such as welding, soldering, brazing, or applying adhesive.

Irrespective of the configuration of the legs 34 (e.g., legs 34' or 34") of the basket 24, those legs are constructed so as to be resistant to laser strikes to avoid damage or destruction of the basket and/or damage to the patient vessel or cavity. Such laser resistance is achieved through use of a high-temperature metal in the construction of the basket legs 34. One class of high-temperature metals that can be used is that of refractory metals, which include niobium, tantalum, molybdenum, tungsten, and rhenium. Such metals have very high melting temperatures, i.e., temperatures greater that 2000 °C. In addition to very high melting temperatures, refractory metals also exhibit superior tensile strength at high temperatures. For example, such metals may exhibit tensile strengths of at least about 69 MPa (10 kilo pounds per square inch) at temperatures over about 1093 °C (2000 °F). Those properties render refractory metals highly resistant to breakage due to thermal shock, such as that created by a laser beam strike.

In some embodiments, one or more of the legs 34 can comprise a wire formed of a single refractory metal in pure form (i.e., non-alloyed). Testing conducted by the inventors has shown that such wires are highly resistant to laser beam strikes. In one test, a round tungsten wire having a diameter of 127 µm (0.005 inches) was subjected to multiple laser beam strikes at the same location of the wire from a holmium YAG laser set to a power setting of 6-8 joules. Despite the repeated strikes, no apparent damage was caused to the wire. It was not until several strikes at a setting of 12 joules (much higher that that normally used in surgical procedures) were delivered to the same point on the wire that breakage was observed. Despite such breakage, it is unlikely that the same point of a wire would be repeatedly struck during an actual surgical procedure. Therefore, the testing indicated that a basket constructed of a refractory metal, such as tungsten, would likely survive one or more laser strikes during surgery without damage or failure.

Although the basket legs 34 can be constructed from wires composed of a single refractory metal, the wires can alternatively be composed of a refractory metal alloy. Such an alloy can comprise an alloy of two or more refractory metals, such as a tungsten-rhenium alloy, or an alloy of a refractory metal and another material (e.g., steel).

Other suitable high-temperature metals include various superalloys. The term "superalloy" refers to a class of metal alloys that have been specifically designed for special applications in which high performance is needed, often under extreme conditions. Suitable superalloys for the construction of the basket 34 comprise high- temperature superalloys that include nickel and/or cobalt, such as, but not limited to, Astroloy, Incoloy, Inconel, Nimonic, Haynes 188, Rene 95, and Udimet.

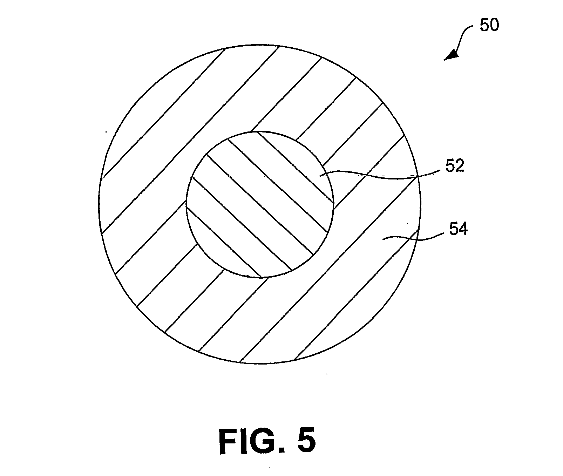

Although desirable laser resistance can be obtained when the legs 34 of the basket 24 are constructed of the high-temperature metals described above alone, the mechanical properties of the high-temperature metals may be less desirable than those of other materials for construction of a basket. Accordingly, the legs 34 of the basket 24 can be constructed as a composite of a high-temperature metal (in pure or alloyed form) and another material. FIGS. 5-8 illustrate example composite wires that can be used to form the basket legs 34.

Beginning with FIG. 5, illustrated is a round wire 50 that can be used to construct the basket 24, and more particularly the basket legs 34, shown in FIGS. 1 and 2. As is indicated in FIG. 5, the wire 50 includes a core 52 and an a coaxial outer layer 54. The core 52 is formed of a high-temperature metal of the type described above. Therefore, the core 52 comprises one or more of a refractory metal (pure or alloyed) or a high-temperature superalloy. The outer layer 54, however, comprises another material that provides the physical attributes that are desired in a retrieval device basket, such as elasticity and memory. Suitable materials for the outer layer 54 include shape-memory materials such as, for example, a nickel and titanium alloy (commonly referred to as "nitinol"). Other suitable shape-memory materials may include one or more of titanium-palladium-nickel, nickel-titanium-cooper, gold-cadmium, iron-zinc-cooper-aluminum, titanium-niobium-aluminum, uranium-niobium, hafnium-titanium-nickel, iron-manganese-silicon, nickel-titanium, nickel-iron-zinc-aluminum, copper-aluminum-iron, titanium-niobium, zirconium-copper-zinc, nickel-zirconium-titanium. Alternatively, another material, such as stainless steel, may be used to construct the outer layer 54.

The core 52 and the outer layer 54 can be combined in a variety of different ways. In some embodiments, the core 52 and the outer layer 54 are co-extruded as a composite wire. In other embodiments, the core 52 comprises a pre-formed solid wire that is encapsulated by a tube that forms the outer layer 54. In such a case, the tube can loosely surround the core 52, or can be bonded to the core, either at discrete points or along the entirety of its length.

In the embodiment of FIG. 5, the non-high temperature material (e.g., nitinol or stainless steel) is provided on the outside of the wire 50 (as opposed to the core) so that the mechanical properties of that material dictate the mechanical properties of the wire. In other words, given that the stresses within the wire 50 are greatest at the outer portions of the wire when the wire is bent (e.g., to form the basket 24), the properties of the material that comprises those outer portions are likely to be dominant for the wire as a whole. As is indicated in FIG. 5, the amount of material of the outer layer 54 can be greater than that of the core 52 to ensure such dominance. Despite that fact, however, the core 52 is large enough to provide the desired laser resistance. Therefore, should the wire 50 be struck by a laser beam, the core 52 of the wire will remain in tact even if the outer layer 54 does not. In such a case, the basket 24 will still be functional and damage to the patient can be avoided.

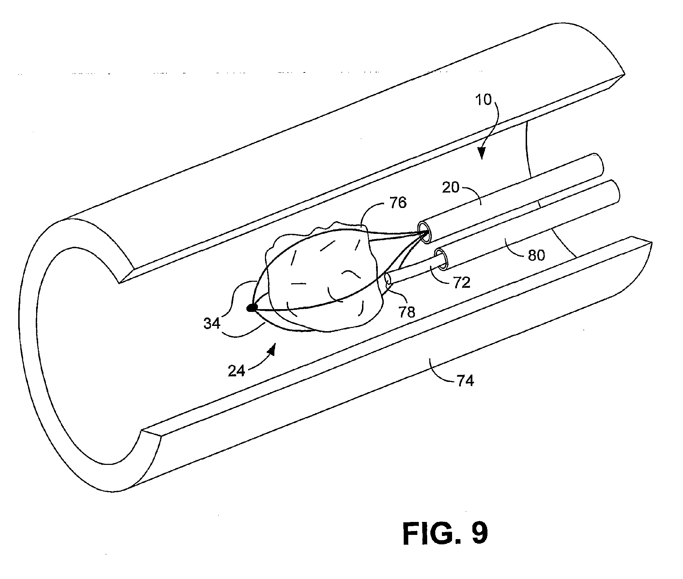

FIG. 9 illustrates use of the laser-resistant retrieval device 10 in conjunction with a laser lithotriptor 72 within a patient vessel 74 (e.g., ureter). As is indicated in FIG. 9, a stone 76 can been captured using the basket 24. While the stone 76 is held in place with the basket 24, the laser lithotriptor 72 can be positioned such that its tip 78 extends into the interior space defined by the basket 24 adjacent the stone 76. By way of example, such positioning can be facilitated by a further instrument 80, such as a catheter or endoscope (e.g., ureteroscope). The laser lithotriptor 72 can then be operated to emit laser beam pulses that fracture the stone 76 into smaller pieces. If during such a process a laser beam strikes a leg 34 of the basket 24, the high-temperature metal of the leg will prevent the leg from breaking such that basket can continue to be used to hold the stone 76 or its fragments in place.

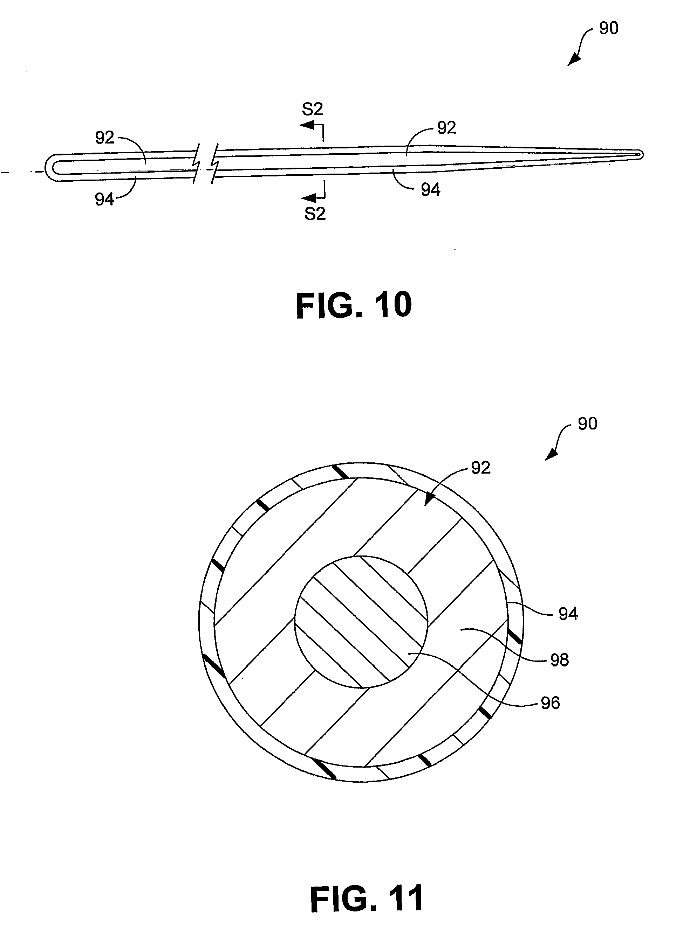

FIGS. 10 and 11 illustrate a first embodiment of a second laser-resistant surgical device 90. The surgical device 90 comprises a guidewire that is designed to facilitate insertion of other surgical devices into a patient vessel or cavity. Such a wire may, for example, be designed for use in the urinary tract or blood vessels. For clarity purposes, the surgical device 90 will be referred to as a guidewire for the discussion of FIGS. 10 and 11.

As is indicated in FIG. 10, the guidewire 90 includes a core wire 92 that is surrounded by an outer layer or jacket 94 of material, such as a polymeric material (e.g., polyurethane or nylon). To increase the laser resistance of the guidewire, the core wire 92 comprises a high-temperature metal. As with the embodiments for the laser-resistant surgical device 10 described above, that high-temperature metal can comprise one or more of a refractory metal (pure or alloyed) or a high-temperature superalloy.

In some embodiments, the core wire 92 only comprises high-temperature metal(s). Alternatively, however, the core wire 92 can be a composite wire having a construction that is similar to that described above in relation to FIG. 5. In such a case, the core wire 92 may comprise a round wire having a core and a coaxial outer layer of disparate material. An example of such a construction is depicted in FIG. 11.

As is indicated in FIG. 11, the core wire 92 comprises a core 96 and an outer layer 98. The core 96 comprises a high-temperature metal such as that described above, and the outer layer 98 comprises another material that has desirable mechanical properties (e.g., nitinol, stainless steel). The presence of the high-temperature metal increases the laser resistance of the guidewire 90 such that the guidewire is less likely to break if struck by a laser beam during a surgical procedure.

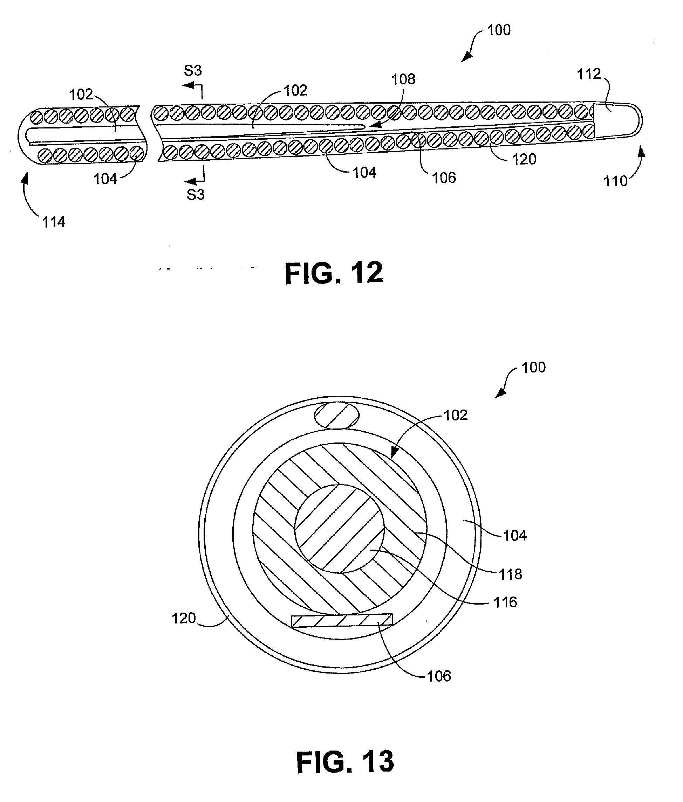

FIGS. 12 and 13 illustrate a second embodiment for the second surgical device, i.e., a guidewire 100. In this embodiment, the guidewire 100 includes a core wire 102, a coiled wire 104 that surrounds the core wire, and a safety wire 106 that is positioned between the core wire and the coiled wire. As is indicated in FIG. 12, the distal end 108 of the core wire 102 is tapered and does not extend to the distal end or tip 110 of the guidewire 100. The coiled wire 104 and the safety wire 106, however, extend to a cap member 112 provided within the tip 110 of the guidewire 100. The coiled wire 104 and the safety wire 106 can be secured together within the cap member 112 using a suitable bonding method including welding, soldering, brazing, or applying adhesive. In addition or in exception, the coiled wire 104 and the safety wire 106 can be secured together at discrete locations along the length of the safety wire, or along its entire length. Moreover, each of the core wire 102, coiled wire 104, and safety wire 106 can be similarly secured together at the proximal end 114 of the guidewire 100.

As is apparent from FIG. 13, the core wire 102 is a composite wire and therefore comprises a core 116 and a coaxial outer layer 118. As with the composite wire 90 of FIG. 11, the core 116 can comprise a high-temperature metal wire and the outer layer 118 can comprise another material (e.g., nitinol, stainless steel), or vice versa. As is further apparent from FIG. 13, the coiled wire 104 can, optionally, be coated with an outer layer 120 of polymeric material, lubricious material, hydrophilic material, and/or other material.

The coiled wire 104 and the safety wire 106 can also be made of a high-temperature metal. In other embodiments, however, at least the coiled wire 104 is constructed of another material that provides the desired column strength to the wire, such as stainless steel.

Irrespective of the particular configuration of the guidewire 100, the presence of the high-temperature metal increases the laser resistance of the guidewire such that, like guidewire 90, the guidewire 100 is less likely to break if struck by a laser beam during a surgical procedure.

From the foregoing, it can be appreciated that laser-resistant surgical devices can be provided that comprise laser-resistant wires. Although retrieval devices and guidewires have been described as specific types of laser-resistant surgical devices, the disclosure generally applies to any surgical device that includes a wire that is exposed within the body during a surgical procedure and is susceptible to a laser beam strike. Therefore, laser-resistant wires similar to those described in the foregoing can be provided in other surgical devices including, for example, graspers, rakes, forceps, and the like.

## Claims

1. A laser-resistant surgical device (10), comprising:
a laser-resistant wire (40) that is exposed when used in the body during a surgical procedure so as to be susceptible to a laser beam strike, **characterized in that**: the wire comprising a high-temperature refractory or superalloy metal that is highly resistant to thermal shock,
and
the wire has a core (52) of said high-temperature metal and a coaxial outer layer (54) of another material, a material that provides the surgical device with elasticity in response to imposed bending stresses.

2. The device of claim 1, wherein the high-temperature metal comprises at least one of niobium, tantalum, molybdenum, tungsten, and rhenium.

3. The device of claim 1, wherein the high-temperature metal comprises an alloy of at least two of niobium, tantalum, molybdenum, tungsten, and rhenium.

4. The device of claim 1, wherein the high-temperature metal comprises a superalloy.

5. The device of claim 1, wherein the wire is a round wire.

6. The device of claim 1, wherein the outer layer is composed of a shape-memory material or stainless steel.

7. The. device of claim 1, wherein the device is a retrieval device and the wire forms a leg of a basket of the device.

8. The device of claim 1, wherein the device is a guidewire and the laser-resistant wire forms a wire of the guidewire.

9. The guidewire of claim 8, wherein the laser-resistant wire is a core wire and the guidewire further comprises a coiled wire that surrounds the core wire and a safety wire that is positioned between the core wire and the coiled wire.

10. The guidewire of claim 9, wherein the coiled wire and the safety wire are composed of stainless steel.

11. The guidewire of claim 9, of which the outer layer is of polymeric material.

## Patentansprüche

1. Laserresistente chirurgische Vorrichtung (10), mit:
einem laserresistenten Draht (40), der bei Verwendung in dem Körper während eines chirurgischen Eingriffs freigelegt ist, um für einen Laserstrahlbeschuss empfänglich zu sein, **dadurch gekennzeichnet, dass** der Draht ein hochtemperaturfestes Metall aufweist oder eine Metallsuperlegierung, die gegen einen Temperaturschock sehr widerstandsfähig ist,
und der Draht einen Kern (52) des Hochtemperaturmetalls und eine koaxiale äußere Lage (54) eines weiteren Materials aufweist, und zwar eines Materials, dass der chirurgischen Vorrichtung als Antwort auf aufgebrachte Biegespannungen Elastizität bereitstellt.

2. Vorrichtung nach Anspruch 1, bei der das Hochtemperaturmetall Niobium, Tantal, Molybdän, Wolfram und/oder Rhenium aufweist.

3. Vorrichtung nach Anspruch 1, bei der das Hochtemperaturmetall eine Legierung aus mindestens zwei von Niob, Tantal, Molybdän, Wolfram und Rhenium aufweist.

4. Vorrichtung nach Anspruch 1, bei der das Hochtemperaturmetall eine Superlegierung aufweist.

5. Vorrichtung nach Anspruch 1, bei welcher der Draht ein runder Draht ist.

6. Vorrichtung nach Anspruch 1, bei der die äußere Lage aus einem Formgedächtnismaterial oder rostfreiem Stahl zusammengesetzt ist.

7. Vorrichtung nach Anspruch 1, wobei die Vorrichtung eine Zurückholvorrichtung ist und der Draht einen Schenkel eines Korbs der Vorrichtung ausbildet.

8. Vorrichtung nach Anspruch 1, wobei die Vorrichtung ein Führungsdraht ist und der laserresistente Draht einen Draht des Führungsdrahts ausbildet.

9. Führungsdraht nach Anspruch 8, bei dem der laserresistente Draht ein Kerndraht ist und der Führungsdraht ferner einen gewundenen Draht aufweist, der den Kerndraht umgibt, und einen Sicherheitsdraht, der zwischen dem Kerndraht und dem gewundenen Draht angeordnet ist.

10. Führungsdraht nach Anspruch 9, bei dem der gewundene Draht und der Sicherheitsdraht aus rostfreiem Stahl zusammengesetzt sind.

11. Führungsdraht nach Anspruch 9, von dem die äußere Lage aus einem Polymermaterial ist.

## Revendications

1. Dispositif chirurgical résistant au laser (10), comprenant :
un fil résistant au laser (40) qui est exposé lorsqu'il est utilisé dans le corps au cours d'une intervention chirurgicale de manière à pouvoir être frappé par un faisceau laser, **caractérisé en ce que** le fil comprenant un métal en superalliage ou réfractaire à haute température qui est hautement résistant au choc thermique,
et
le fil possède un noyau (52) dudit métal à haute température et une couche extérieure coaxiale (54) d'un autre matériau, un matériau qui fournit de l'élasticité au dispositif chirurgical en réponse à des efforts de flexion imposés.

2. Dispositif de la revendication 1, dans lequel le métal à haute température comprend au moins un élément parmi le niobium, le tantale, le molybdène, le tungstène, et le rhénium.

3. Dispositif de la revendication 1, dans lequel le métal à haute température comprend un alliage d'au moins deux éléments parmi le niobium, le tantale, le molybdène, le tungstène, et le rhénium.

4. Dispositif de la revendication 1, dans lequel le métal à haute température comprend un superalliage.

5. Dispositif de la revendication 1, dans lequel le fil est un fil rond.

6. Dispositif de la revendication 1, dans lequel la couche extérieure est composée d'un matériau à mémoire de forme ou de l'acier inoxydable.

7. Dispositif de la revendication 1, dans lequel le dispositif est un dispositif d'extraction et le fil forme une patte d'un panier du dispositif.

8. Dispositif de la revendication 1, dans lequel le dispositif est un fil guide et le fil résistant au laser forme un fil du fil guide.

9. Fil guide de la revendication 8, dans lequel le fil résistant au laser est un fil central et le fil guide comprend en outre un fil enroulé qui entoure le fil central et un fil de sécurité qui est positionné entre le fil central et le fil enroulé.

10. Fil guide de la revendication 9, dans lequel le fil enroulé et le fil de sécurité sont composés d'acier inoxydable.

11. Fil guide de la revendication 9, dont la couche extérieure est réalisée en un matériau polymère.
